# EUROPEAN PATENT APPLICATION

(11) **EP 1 870 016 A1**
(43) Date of publication of application: **26.12.2007**
(21) Application number: 06731439.3
(22) Date of filing: 07.04.2006
(51) Int. Cl.: A61B 1/00

(54) **ELECTRICALLY CURVABLE ENDOSCOPE DEVICE**

(30) Priority: 11.04.2005 JP 2005113921
(71) Applicant: Olympus Corporation, Tokyo 151-0072 (JP)
(72) Inventor: IKEDA, Yuichi, Olympus Medical Systems Corp., Tokyo 151-0072 (JP); FURUKAWA, Tatsuya c/o Olympus Medical Systems Corp., Tokyo 151-0072 (JP); KOITABASHI, Masanobu c/o Olympus Medical Corp., Tokyo 151-0072 (JP); UENO, Haruhiko c/o Olympus Medical Corp., Tokyo 151-0072 (JP); KANAZAWA, Noriaki c/o Olympus Medical Systems Corp., Tokyo 151-0072 (JP); ISHIZUKA, Tatsuya c/o Olympus Medical Systems Corp., Tokyo 151-0072 (JP); MASAKI, Yutaka c/o Olympus Medical Systems Corp., Tokyo 151-0072 (JP)
(74) Representative: von Hellfeld, Axel
(86) International application number: PCT/JP2006/307492
(87) International publication number: WO 2006/109745

(57) **Abstract**

The present invention has an object to provide an electric bending endoscope apparatus with a configuration which is always capable of securing favorable operability at a time of using an endoscope without placing a weight burden on an operator when operating the endoscope, and for the purpose, the electric bending endoscope apparatus includes at least an electric bending endoscope that is configured by an elongated insertion portion which is inserted into a body cavity, a bending drive section which electrically drives a part of the insertion portion to bend, and an operation portion configured to be separate from the bending drive section and electrically connected to the bending drive section, and performs observation and treatment of an inside of the body cavity, a system controller which is electrically connected to the electric bending endoscope and centrally controls the electric bending endoscope, and an endoscope holding device configured by a bending drive unit which contains the bending drive section and holds a part of the electric bending endoscope, and an arm portion which is connected to the bending drive unit and holds the bending drive unit, and is configured such that by the bending drive unit being attachably and detachably connected to a proximal end side of the insertion portion, the electric bending endoscope and the bending drive section are electrically connected.

## Description

### Technical Field

The invention relates to an electric bending endoscope apparatus, and more particularly to an electric bending endoscope apparatus including an endoscope holding device holding an electric bending endoscope which electrically drives a bending portion provided at an insertion portion of the endoscope to bend.

### Background Art

Conventionally, endoscopes configured to be able to observe organs of insides of body cavities by inserting elongated insertion portions into the body cavities, and to be able to perform various medical treatments by using treatment instruments inserted in treatment instrument channels of the insertion portions as required have come into wide use.

Such a conventional endoscope is generally configured by providing a bending portion configured to be able to bend vertically and laterally at a distal end side of the insertion portion. A bending wire which is extended from an operation portion side is connected to a predetermined region of the bending portion, so that the bending portion is configured to be able to bend in a desired direction by performing a pulling operation and a loosening operation of the bending wire by using an operation member at the operation portion side.

The bending wire in the conventional endoscope has been generally operated mechanically by manually operating the operation member provided at the operation portion. However, in recent years, there have been made various proposals about electric bending endoscope apparatuses configured to perform pulling operations of bending wires by using electric bending drive means such as electric motors.

Since in an endoscope in such an electric bending endoscope apparatus, the electric bending drive means such as an electric motor is disposed at an operation portion or the like, the operation portion tends to increase in size and its weight also tends to increase. Accordingly, when performing a bending operation by using an operation member provided at the operation portion while holding the operation portion with one hand, the load exerted on the user tends to be large. Therefore, the operation becomes difficult to perform.

Thus, for the above reason, in an electric bending endoscope apparatus, as a contrivance for reducing the load exerted on a user when using the apparatus, a proposal about an endoscope holding device for holding an operation portion of an endoscope during use has been conventionally made by, for example, Japanese Patent Laid-Open No. 63-122416 and the like.

The endoscope holding device disclosed by the above described Japanese Patent Laid-Open No. 63-122416 is configured to be provided in a predetermined position such as a wall surface of an endoscope inspection room, and hold an endoscope in use to thereby reduce a burden on an operator (user) during use of the endoscope and secure enhancement in operability.

Meanwhile, for the purpose of enhancement in operability in the conventional electric bending endoscope apparatus, various endoscope apparatuses each with the configuration in which electric bending drive means such as an electric motor, for example, is provided as a separate unit from an operation portion, and the bending drive unit and the operation portion provided separately from the bending drive unit are connected by a cable or the like, whereby the insertion portion and the operation portion can be disposed separately are proposed in, for example, Japanese Patent Laid-Open No. 7-8442, Japanese Patent Laid-Open No. 2002-224016 and the like.

However, according to the means disclosed by the above described Japanese Patent Laid-Open No. 63-122416, the above described Japanese Patent Laid-Open No. 2002-224016 and the like, there arises the problem that the configuration of the endoscope holding device becomes large-scale.

There is the description that each of the electric bending endoscope apparatuses disclosed by the above described Japanese Patent Laid-Open No. 7-8442, the above described Japanese Patent Laid-Open 2002-224016 and the like is configured by arranging various members configuring the bending drive means such as an electric motor inside the holding portion of the endoscope holding device and a control unit, for example, but there is no description of the concrete disposition.

The present invention is made in view of the above described point, and its object is to provide an electric bending endoscope apparatus configured to be always able to secure favorable operability at the time of using an endoscope by being configured so that a weight burden is not exerted on an operator (user) and an bending motor and operation means do not inhibit operability of the endoscope when performing operation of the endoscope.

### Disclosure of the Invention

### Means for Solving the Problem

In order to attain the above described object, an electric bending endoscope apparatus according to the present invention includes at least an electric bending endoscope that is configured by an elongated insertion portion which is inserted into a body cavity, bending drive means which electrically drives a part of the insertion portion to bend, and an operation portion electrically connected to the bending drive means, and can perform observation and treatment of an inside of the body cavity, a system controller which is electrically connected to the electric bending endoscope and centrally controls the electric bending endoscope, and an endoscope holding device configured by a bending drive unit which contains the bending drive means and holds a part of the electric bending endoscope, and an arm portion which is connected to the bending drive unit and holds the bending drive unit, and is configured such that by a proximal end side of the insertion portion being attachably and detachably connected to the bending drive unit, the electric bending endoscope and the bending drive means contained in the bending drive unit are connected.

### Brief Description of the Drawings

Fig. 1 is a block diagram showing an outline of an entire configuration of an electric bending endoscope apparatus of a first embodiment of the present invention;
Fig. 2 is an external perspective view showing the electric bending endoscope apparatus of Fig. 1;
Fig. 3 is an essential part enlarged sectional view showing details of a connecting region of an endoscope insertion portion and a holding device in the electric bending endoscope apparatus of Fig. 1;
Fig. 4 is an external perspective view showing an electric bending endoscope apparatus of a second embodiment of the present invention;
Fig. 5 is an external perspective view showing an electric bending endoscope apparatus of a third embodiment of the present invention;
Fig. 6 is an external perspective view showing only an operation portion and its vicinity in the electric bending endoscope apparatus of Fig. 5 by taking out only an operation portion and its vicinity;
Fig. 7 is an external block diagram showing an electric bending endoscope apparatus of a third embodiment of the present invention;
Fig. 8 is an essential part enlarged perspective view showing details of a connecting region of an endoscope insertion portion and a holding device in the electric bending endoscope apparatus of Fig. 1; and
Fig. 9 is an essential part enlarged view schematically showing the connecting region of the endoscope insertion portion and the holding device in the electric bending endoscope apparatus of Fig. 1 by enlarging the connecting region, and showing a part of an internal configuration of a bending drive unit.

### Best Mode for Carrying Out the Invention

Hereinafter, the present invention will be described in accordance with embodiments shown in the drawings.

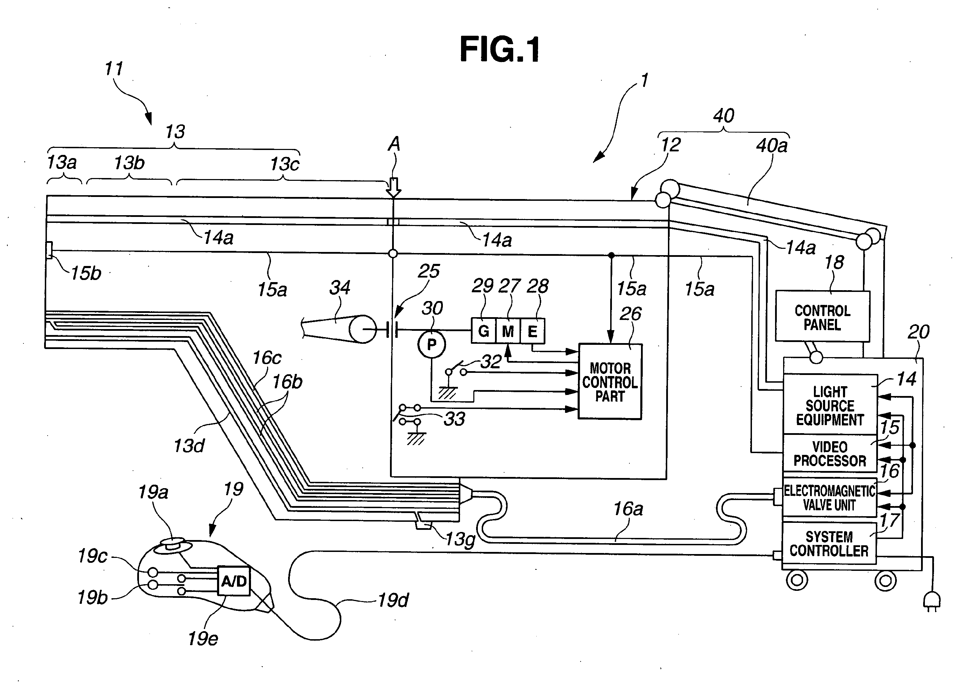

As shown in Fig. 1, an electric bending endoscope apparatus 1 of the present embodiment is mainly configured by an electric bending endoscope 11 that is an endoscope configured by an elongated insertion portion 13 which is inserted into a body cavity, electric bending drive means, an operation portion 19 and the like, and including an observation function and a treatment function of the inside of the body cavity, light source equipment 14 which supplies illumination luminous flux irradiated toward a distal end portion front surface of the insertion portion 13, a video processor 15 which receives a video signal from an image pickup unit 15b provided at a distal end of the insertion portion 13 and performs predetermined signal processing, an electromagnetic valve unit 16 which performs control of air and water supply and suction operation through an air and water supply pipeline 16b, a suction pipeline 16c and the like provided inside the insertion portion 13, a system controller 17 which performs drive control of a bending drive unit 12, and performs centralized control of the light source equipment 14, the video processor 15, the electromagnetic valve unit 16 and the like, a control panel 18 which includes a display portion which receives a video signal from the video processor 15 and displays a predetermined endoscope image and can input various operation instructions by using an operation portion provided on a display surface of the display portion, a cart 20 configured to include moving means such as, for example, a caster to be movable on a floor with the light source equipment 14, the video processor 15, the electromagnetic valve unit 16, the system controller 17, the control panel 18 and the like housed therein and mounted thereon, an endoscope holding device 40 constituted of the bending drive unit 12 which is constituted to contain the bending drive means configuring a part of the electric bending endoscope 11 and attachably and detachably connects to a proximal end side of the insertion portion 13 of the above described endoscope 11, and an arm portion 40a which holds the above described electric bending endoscope 11 in a predetermined position by having its proximal end side fixed to the cart 20 and having a proximal end of the above described bending drive unit 12 connected to its distal end side and the like, an operation portion 19 which is configured to be separate from the above described bending drive unit 12 and is electrically connected to the bending drive unit 12 via the system controller 17, and the like.

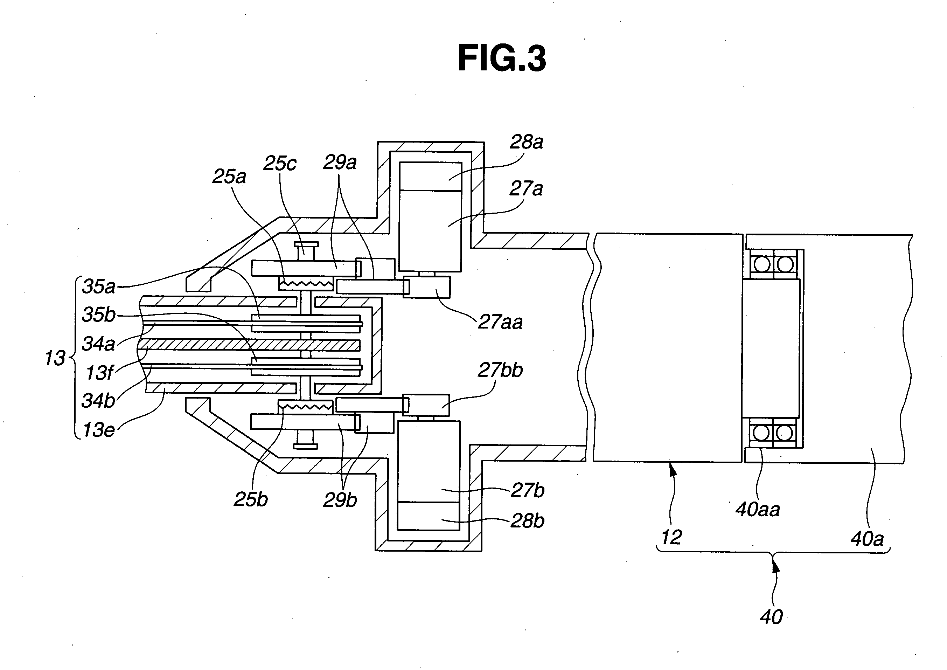

The insertion portion 13 and the bending drive unit 12 are attachable and detachable at the attaching and detaching region shown by reference character A in Fig. 1 via engaging means (25). As the engaging means (25), for example, a jaw clutch (see reference numerals 25a and 25b in Fig. 3) or the like is applied. By the engaging means (25), the insertion portion 13 and the bending drive unit 12 are attachably and detachably connected, and when both of them are in a connected state, the drive force from the bending drive unit 12 is transmitted to the insertion portion 13 side.

The detailed configuration of the connecting region of the insertion portion 13 configuring a part of the electric bending endoscope 11, and the bending drive unit 12 and the arm portion 40a configuring a part of the endoscope holding device 40 will be described later (see Fig. 3).

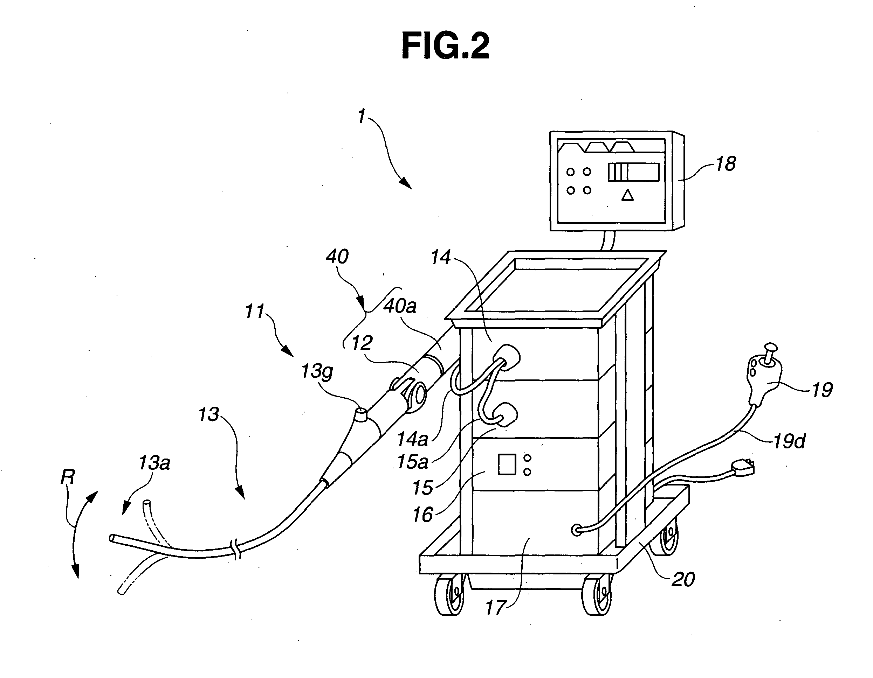

The insertion portion 13 is configured by a distal end rigid portion 13a which contains the image pickup unit 15b configured by an image pickup optical system (not shown), an image pickup device such as a CCD and the like, and is formed at the most distal end side, a bending portion 13b which is connected to a proximal end side of the distal end rigid portion 13a and is configured to be able to operate to bend vertically and laterally (for example, the arrow R direction in Fig. 2 and the like) by drive control of the bending drive unit 12 connected via the system controller 17 in response to the bending operation instruction by the operation portion 19, and a flexible tube portion 13c connected to a proximal end side of the bending portion 13b and formed into an elongated shape.

An angle wire 34 (described later. See also Fig. 3) driven by receiving a drive force from the bending drive unit 12 is inserted through the insertion portion 13. The angle wire 34 is connected to a distal end side of the bending portion 13b (the connection region is not especially illustrated). Thereby when the angle wire 34 is driven by receiving the drive force from the bending drive unit 12, the bending portion 13b can be bent in the vertical and lateral direction (for example, the arrow R direction in Fig. 2 and the like).

Further, the air and water supply pipeline 16b and the suction pipeline 16c are inserted through the insertion portion 13 as described above. An air and water supply port and a suction port are opened in distal end side front surfaces of the air and water supply pipeline 16b and the suction pipeline 16c (not especially illustrated).

A forceps pipeline 13d through which a treatment instrument such as a forceps is inserted is inserted through the insertion portion 13. A forceps port is opened in a distal end side front surface of the forceps pipeline 13d (not especially illustrated). A proximal end side of the forceps pipeline 13d communicates with a forceps insertion port 13g formed in the vicinity of the attaching and detaching region A of the above described bending drive unit 12. Thereby, the treatment instrument such as a forceps which is inserted from the forceps insertion port 13g is inserted through the forceps pipeline 13d and can be protruded from the distal end side front surface of the insertion portion 13.

The bending drive unit 12 is a configuration unit containing an electric motor 27 (see reference numerals 27a and 27b in Fig. 3) for bending drive, and bending drive means such as various members which are formed to transmit and cut off the power generated from the electric motor 27.

More specifically, the bending drive unit 12 is configured by the electric motor 27 (see reference numerals 27a and 27b in Fig. 3) which generates a drive force, a motor control part 26 which performs centralized control of the bending drive unit 12 including the electric motor 27, an encoder 28 (see reference numerals 28a and 28b in Fig. 3) which converts the operation state such as the rotation speed and rotation quantity of the electric motor 27 into data, a reduction gear 29 (see reference numerals 29a and 29b in Fig. 3) which reduces the rotational force of the electric motor 27, a clutch 25 (see reference numerals 25a and 25b in Fig. 3) that is engaging means connected to the reduction gear 29 and transmits the rotational force of the electric motor 27 to the insertion portion 13 side, a potentiometer 30 that is rotational position detecting means, a clutch state detecting switch 32 which detects the state of the clutch 25, an attaching and detaching state detecting switch 33 which detects the engaging state of the insertion portion 13 and the bending drive unit 12, and the like.

A light guide 14a is connected to the light source equipment 14. The light guide 14a is disposed to be inserted through the insides of the arm portion 40a, the bending drive unit 12 and the insertion portion 13 from the light source equipment 14 to extend to the distal end portion of the insertion portion 13. In order to avoid complication of the drawing in Fig. 1, the light guide 14a from the light source equipment 14 to the bending drive unit 12 is illustrated outside the arm portion 40a, but the light guide 14a is actually inserted through the inside of the arm portion 40a as described above.

In this case, the light guide 14a can be separated at the attaching and detaching region A of the bending drive unit 12 and the insertion portion 13, and the light guide 14a is disposed to communicate with each other when both of them are engaged with each other. Thereby, the illumination luminous flux supplied from the light source equipment 14 is irradiated toward the front surface from the distal end portion of the insertion portion 13 via the light guide 14a.

A signal cable 15a which transmits a video signal from the image pickup unit 15b is connected to the video processor 15. The signal cable 15a extends from the image pickup unit 15b at the distal end of the insertion portion 13, is inserted through the insides of the insertion portion 13 and the bending drive unit 12, and further inserted through the inside of the arm portion 40a, and thereafter, is connected to a predetermined terminal of the video processor 15. As for the signal cable 15a, as in the case of the above described light guide 14a, the signal cable 15a from the bending drive unit 12 to the video processor 15 is illustrated outside the arm portion 40a in order to avoid complication of the drawing in Fig. 1, but the signal cable 15a is actually inserted through the inside of the arm portion 40a as described above.

In this case, the signal cable 15a can be separated at the attaching and detaching region A of the bending drive unit 12 and the insertion portion 13, and is electrically connected in the state in which both of them are engaged with each other. Therefore, connection connectors or the like (not especially illustrated) are provided at the bending drive unit 12 and the insertion portion 13 in the attaching and detaching region A of the bending drive unit 12 and the insertion portion 13.

The signal cable 15a electrically connects the motor control part 26 and the system controller 17 and the like via the video processor 15. Thereby, instruction signals from the system controller 17 can be transmitted to the motor control part 26.

The control panel 18 is electrically connected to the video processor 15. Thereby, the video signal outputted from the video processor 15 is transmitted to the control panel 18. On receiving this, the control panel 18 displays a predetermined endoscope image by using the display portion.

A tube 16a connected to the air and water supply pipeline 16b and the suction pipeline 16c of the insertion portion 13 is connected to the electromagnetic valve unit 16. Thereby, the electromagnetic valve unit 16 communicates with the distal end of the insertion portion 13 through the air and water supply pipeline 16b or the suction pipeline 16c, and the tube 16a, and can perform air and water supply and suction from the distal end surface of the insertion portion 13 when the electromagnetic valve unit 16 is driven, and air and water supply and suction operations are performed.

The operation portion 19 includes various operation members which generate a bending operation instruction, air and water supply and suction operation instruction signals, and is configured to be separate from the bending drive unit 12 and the like.

Describing in more detail, the operation portion 19 is configured by various operation members such as an operation stick 19a for performing a bending operation instruction, an air and water supply operation button 19b for performing an air and water supply operation instruction, and a suction operation button 19c performing a suction operation instruction, an AD converter 19e electrically connected to the various operation members (19a, 19b, 19c) and the like. The AD converter 19e performs AD conversion processing of receiving electrical signals generated from the various operation members (19a, 19b, 19c) and converting the electrical signals into predetermined operation instruction signals.

The operation portion 19 is electrically connected to the system controller 17 by an electric cable 19d. Accordingly, the operation instruction signals generated by the above described AD converter 19e are transmitted to the system controller 17 via the electric cable 19d.

The system controller 17 establishes electrical connection with the light source equipment 14, the video processor 15, the electromagnetic valve unit 16 and the control panel 18. The electric cable 19d extending from the operation portion 19 is connected to the system controller 17. Thereby, various instruction signals generated by the respective operation members of the operation portion 19 being operated are transmitted to the system controller 17 through the electric cable 19d. Receiving this, the system controller 17 properly transmits a control signal for performing control corresponding to the instruction signal to each of the devices.

The system controller 17 receives various operation instruction signals from the operation portion of the control panel 18, and properly transmits a control signal for performing control corresponding to each of the instruction signals to each of the devices.

The endoscope holding device 40 is configured by the arm portion 40a fixed to the cart 20 at its proximal end side, the bending drive unit 12 with its proximal end connected to a distal end side of the arm portion 40a, and the like, as shown in Figs. 1 and 2. The insertion portion 13 of the electric bending endoscope 11 is fixedly held at a distal end side of the bending drive unit 12.

In the endoscope holding device 40, the connection region of the arm portion 40a and the bending drive unit 12 is as shown in Fig. 3. Specifically, a rotary support mechanism 40aa such as a bearing is placed at the distal end portion of the arm portion 40a. The proximal end of the bending drive unit 12 is fitted in the rotary support mechanism 40aa. Thereby, the bending drive unit 12 is rotatable around an axis with respect to the arm portion 40a.

Accordingly, the electric bending endoscope 11 is used with the insertion portion 13 and the bending drive unit 12 connected to each other, but when torsion or the like is applied to the insertion portion 13 at the time of its use, the bending drive unit 12 connected to the insertion portion 13 can be rotated in the same direction as the insertion portion 13, and freely and smoothly with respect to the arm portion 40a, by the rotary support mechanism 40aa.

Inside the bending drive unit 12, the electric motor 27 (see reference numerals 27a and 27b in Fig. 3), and various members and the like which are formed to transmit and cut off the power generated from the electric motor 27 are respectively placed at predetermined regions.

The electric motor 27 is constituted of two motors that are a UD motor 27a which contributes to bending drive in the vertical direction (an up (U) direction and a down (D) direction) of the bending portion 13b, and an RL motor 27b which contributes to bending drive in the lateral direction (a right (R) direction and a left (L) direction) of the bending portion 13b.

The encoder 28 is placed at the electric motor 27. In this case, a UD encoder 28a is placed at the UD motor 27a, and an RL encoder 28b is placed at the RL motor 27b respectively. Thereby, the encoders 28a and 28b convert the operation states such as the respective rotation speeds and rotation amounts of the UD motor 27a and the RL motor 27b into data and output the data.

Drive gears 27aa and 27bb are respectively fixed to drive shafts of the UD motor 27a and the RL motor 27b as shown in Fig. 3. The drive gears 27aa and 27bb are meshed with the reduction gear 29 constituted of a two speed gear and a flat gear (a UD reduction gear 29a and an RL reduction gear 29b). The flat gear of the reduction gear 29 is fixed to a rotary shaft 25c of the clutch 25 (a UD clutch 25a, an RL clutch 25b).

The rotary shaft 25c is disposed by penetrating through a casing 13e configuring a part of the insertion portion 13 of the electric bending endoscope 11 and a frame portion 13f internally provided near the proximal end portion of the insertion portion 13. Outside the casing 13e, respective meshing portions of the UD clutch 25a and the RL clutch 25b in the clutch 25 are fixed to both ends of the rotary shaft 25c.

Inside the casing 13e, a UD drum 35a and an RL drum 35b are fixed to the rotary shaft 25c. The UD drum 35a and the RL drum 35b rotate with rotation of the rotary shaft 25c.

As a result, when the electric motor 27 (27a, 27b) normally and reversely rotates, its rotational drive force normally and reversely rotates the drive shaft to rotate the drive gears 27aa and 27bb normally and reversely. The drive force is transmitted to the rotary shaft 25c from the drive gears 27aa and 27bb through the reduction gear 29 (29a, 29b). When the rotary shaft 25c normally and reversely rotates, the UD drum 35a and the RL drum 35b also rotate normally and reversely. Receiving this, the angle wire 34 (a UD angle wire 34a and an RL angle wire 34b) is each pulled in a predetermined direction. Thereby, the angle wire 34 acts on the bending portion 13b of the distal end rigid portion 13a of the insertion portion 13, so that the bending operation in the vertical direction or in the lateral direction of the bending portion 13b is performed.

The bending drive unit 12 and the insertion portion 13 are detachable and attachable by engagement and disengagement of the meshing portions of the clutch 25. Thereby, after use of the electric bending endoscope 11, for example, the insertion portion 13 is detached from the bending drive unit 12, and only the insertion portion 13 can be cleaned.

As described above, according to the above described first embodiment, the electric bending endoscope apparatus is configured such that the bending drive unit 12 internally provided with the electric motor 27 for bending drive, drive force transmitting members and the like in the electric bending endoscope 11 is integrally connected to the arm portion 40a configuring a part of the endoscope holding device 40, and the insertion portion 13 of the electric bending endoscope 11 is connected to the distal end side of the bending drive unit 12. Since the electric bending endoscope 11 is supported by the endoscope holding device 40, and the bending drive unit 12 is configured as a part of the endoscope holding device 40, the heavy bending drive unit 12 is reliably supported by the endoscope holding device 40. Thereby, an operator (user) has only to operate the insertion portion 13, the operation portion 19 and the like with small weight. Therefore, reduction in the weight burden on the operator (user) can be realized, and favorable operability can be provided.

Since the bending drive unit 12 including the electric motor 27 and the like is configured to be included in the endoscope holding device 40, the present invention can contribute to reduction in the space for disposition of the members, and can contribute to reduction in size as the electric bending endoscope apparatus 1.

All the devices configuring the electric bending endoscope apparatus 1 are configured to be able to be loaded on the cart 20 including the moving means such as a caster, and therefore, a complete set of the electric bending endoscope apparatus 1 can be easily moved.

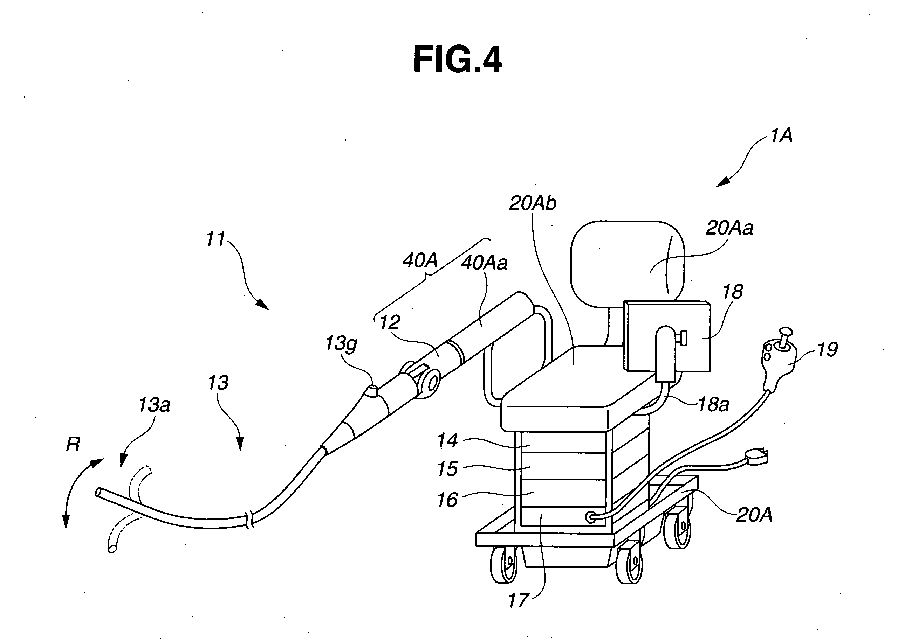

Next, an electric bending endoscope apparatus of a second embodiment of the present invention will be described hereinafter by using Fig. 4.

The basic configuration of the present embodiment is substantially the same as that of the above described first embodiment. The present embodiment differs from the first embodiment in the respect that the present embodiment is configured such that an operator (user) can carry out inspection, treatment action and the like in a seated state when using an electric bending endoscope apparatus 1A of the embodiment by providing a seat plate portion 20Ab and a seat back rest portion 20Aa on the top surface of a cart 20A as shown in Fig. 4.

In this case, an endoscope holding device 40A constituted of an arm portion 40Aa and the bending drive unit 12 connected to the arm portion 40Aa is placed at a region corresponding to an armrest of the cart 20A which functions as a chair. The endoscope holding device 40A in the present embodiment is disposed at the right-hand side in the state in which the operator is seated in the cart 20A as shown in Fig. 4.

Meanwhile, in the cart 20A, at the side opposite from the side at which the endoscope holding device 40A is placed, the control panel 18 fixedly supported by an arm 18a extending from an undersurface side of the seat plate portion 20Ab is disposed. The control panel 18 is electrically connected to the video processor 15 and the system controller 17 which are provided at a portion under the seat plate portion 20Ab of the cart 20A. The other configuration is substantially the same as that of the above described first embodiment.

As described above, according to the above described second embodiment, the same effect as in the above described first embodiment can be obtained. In addition, in the present embodiment, a seat back rest portion 21Aa and a seat plate portion 21 Ab are placed so that the operator can be seated therein by using a top surface space of the cart 20A which is configured to be movable and is loaded with all the devices configuring the electric bending endoscope apparatus 1A. The endoscope holding device 40A is placed at the region to be the armrest. Thereby, the operator can easily perform inspection, treatment action and the like using the electric bending endoscope (11) in a seated state and in a more comfortable posture without a weight burden exerted on the operator.

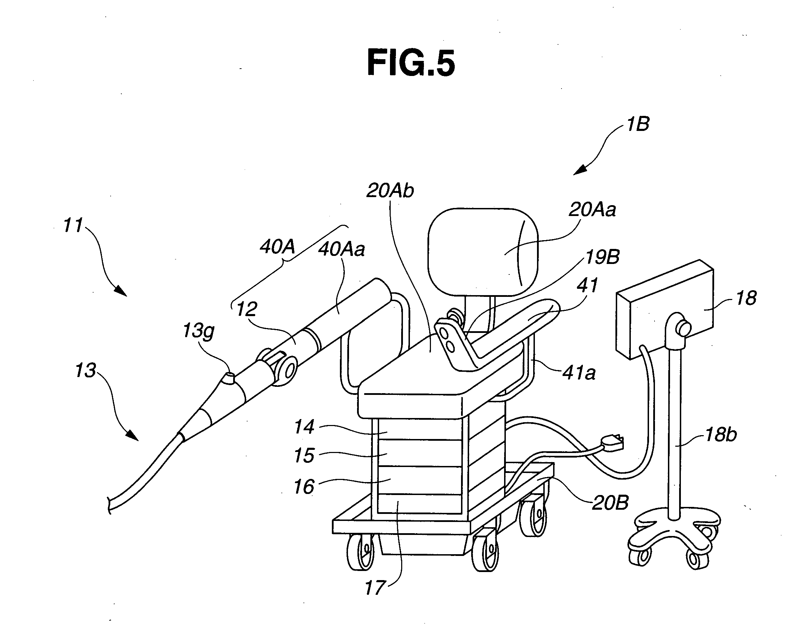

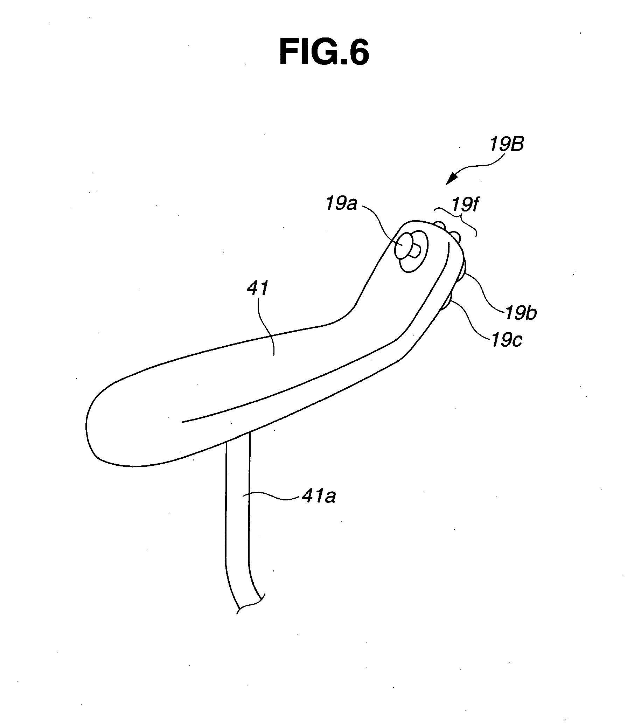

Next, an electric bending endoscope apparatus of a third embodiment of the present invention will be described hereinafter by using Figs. 5 and 6.

The basic configuration of the present embodiment is substantially the same as that in the above described second embodiment. The present embodiment differs from the second embodiment in the respect that in the present embodiment, an operation portion 19B including various operation members which generate operation instructions such as a bending operation instruction, air and water supply and suction operation instruction signals is placed at one (see reference numeral 41) of the regions corresponding to the armrests of a cart 20B which functions as a chair as shown in Fig. 5. With this, in the example shown in Fig. 5, the control panel 18 is placed separately by a dedicated leg 18b. However, disposition of the control panel 18 is not limited to this. For example, totally similarly to the above described second embodiment, the configuration in which an arm (18a; see Fig. 4) is extended from the cart 20B, and the control panel 18 is fixedly supported by the arm (18a) may be adopted.

On the other side of the armrest region (the right hand side in the state in which the operator is seated in the cart 20B in, for example, Fig. 5), the endoscope holding device 40A constituted of the arm portion 40Aa and the bending drive unit 12 connected to the arm portion 40Aa as in the above described second embodiment is placed.

In the operation portion 19B, various operation members, that is, the various operation members such as the operation stick 19a for performing a bending operation instruction, the air and water supply operation button 19b for performing an air and water supply operation instruction, the suction operation button 19c for performing a suction operation instruction, and a system control button 19f are respectively placed at predetermined regions, at a distal end side of the armrest portion 41 at the left hand side in the state in which the operator is seated in the cart 20B as described above. The various operation members (19a, 19b, 19c, 19f) are electrically connected to the AD converter (not illustrated in Figs. 5 and 6. See reference numeral and character 19e in Fig. 4) placed inside the operation portion 19B of the left armrest portion 41. The output signal from the AD converter is outputted to the system controller 17 through a predetermined signal cable (not illustrated) which is inserted through the inside of the left armrest portion 41.

Thereby, when the operator (user) uses the electric bending endoscope apparatus 1B, the operator (user) can carry out inspection, treatment action and the like in the seated state. The other configuration is substantially the same as that of the above described second embodiment.

As described above, according to the above described third embodiment, the same effect as in the above described second embodiment can be obtained. In addition, in the present embodiment, the configuration in which the operation portion 19B is also placed to be integrated with the armrest portion 41 is adopted, the present embodiment can contribute to further enhancement in operability.

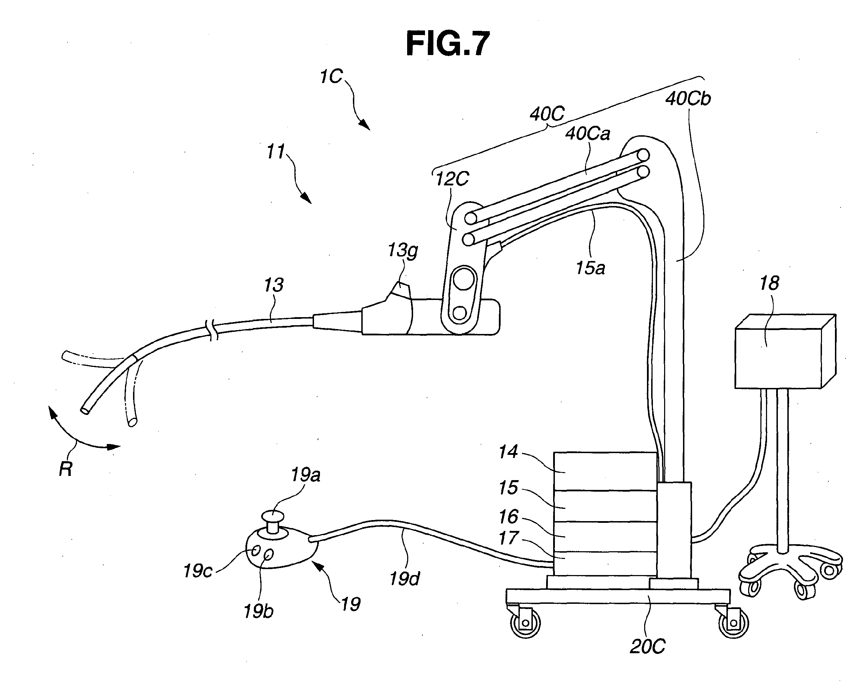

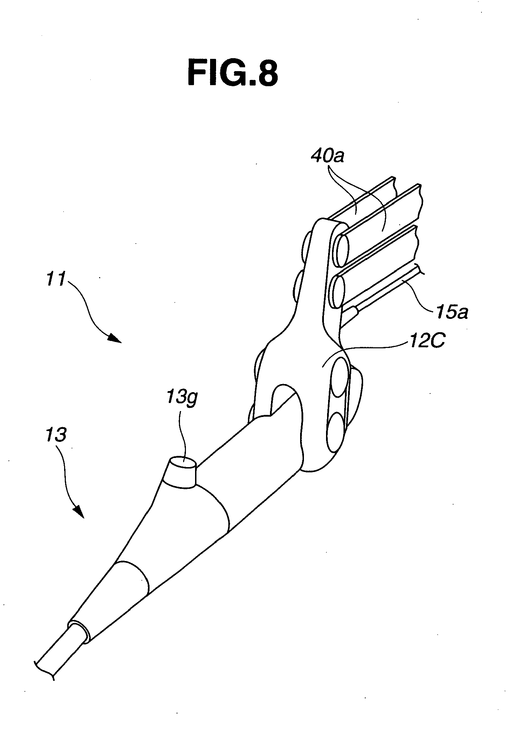

Next, an electric bending endoscope apparatus of a fourth embodiment of the present invention will be described hereinafter by using Figs. 7 to 9.

The basic configuration of the present embodiment is substantially the same as that of the above described first embodiment. The present embodiment slightly differs from the first embodiment in the configuration of an endoscope holding device 40C as shown in Fig. 7. Specifically, in an electric bending endoscope apparatus 1C of the present embodiment, the endoscope holding device 40C is configured by a support 40Cb integrally fixed to a cart 20C to be upright, a bending drive unit 12C containing the electric motor 27 and the like, a parallel link 40Ca which is configured to connect the bending drive unit 12C and the above described support 40Cb and can move the electric bending endoscope 11 connected to the bending drive unit 12C in a predetermined direction (longitudinal and vertical directions) and the like.

The support 40Cb and the parallel link 40Ca in the present embodiment are members corresponding to the arm portion (40a, 40Aa) in each of the above described embodiments.

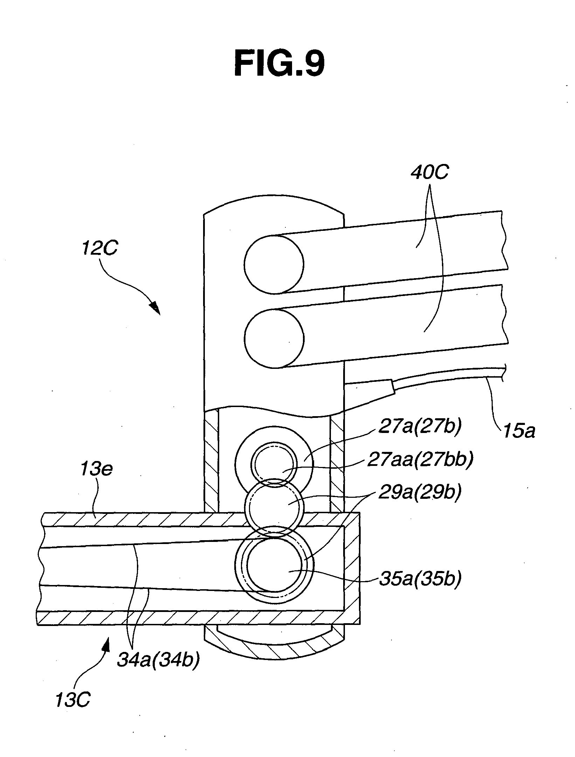

The electric motor 27 (27a, 27b; see Fig. 9), the drive gear (27aa, 27bb) fixed to the rotary shaft of the electric motor 27, the reduction gear 29 (29a, 29b; see Fig. 9) and the like are placed inside the bending drive unit 12C as in each of the above described embodiments. The drums 35a and 35b, the angle wire 34 (34a, 34b) and the like are placed inside the casing 13e of the insertion portion 13 as in each of the above described embodiments.

Further, the bending drive unit 12C, and the video processor 15, the system controller 17 and the like are electrically connected by the signal cable 15a. The other configuration is substantially the same as that of the above described first embodiment.

As described above, according to the above described fourth embodiment, the same effect as in the above described first embodiment can be obtained. In addition, the endoscope holding device 40C in the present embodiment can hold the bending drive unit 12C at a higher position by the support 40Cb and the parallel link 40Ca, while the position of the bending drive unit 12C which is held is made movable in the back and forth and vertical directions, and therefore, more favorable operability can be obtained when using the electric bending endoscope 11.

The present application is applied with Japanese Patent Application No. 2005-113921 filed in Japan on April 11, 2005 as the basis of claim of priority.

The above described disclosure content is the same content cited in the specification, claims, and the drawings of the present application.

## Claims

1. An electric bending endoscope apparatus comprising at least:
an electric bending endoscope that is configured by an elongated insertion portion which is inserted into a body cavity, bending drive means which electrically drives a part of the insertion portion to bend, and an operation portion electrically connected to the bending drive means, and can perform observation and treatment of an inside of the body cavity;
a system controller which is electrically connected to the electric bending endoscope and centrally controls the electric bending endoscope; and
an endoscope holding device configured by a bending drive unit which contains the bending drive means and holds a part of the electric bending endoscope, and an arm portion which is connected to the bending drive unit and holds the bending drive unit,
wherein by a proximal end side of the insertion portion being attachably and detachably connected to the bending drive unit, the electric bending endoscope and the bending drive means contained in the bending drive unit are connected.

2. The electric bending endoscope apparatus according to claim 1, further comprising:
a cart loaded with at least the system controller,
wherein the endoscope holding device is fixed to a part of the cart.

3. The electric bending endoscope apparatus according to claim 2,
wherein the cart is formed into a mode in a chair shape having a seat back rest portion, a seat plate portion, and an armrest portion, and
the arm portion of the endoscope holding device is formed to function as the armrest portion of the cart, and the bending drive unit is connected to one end of the arm portion.

4. The electric bending endoscope apparatus according to claim 3,
wherein the operation portion is formed integrally with respect to the armrest portion of the cart.
